# EUROPEAN PATENT SPECIFICATION

(11) **EP 4 110 880 B1**
(45) Date of publication and mention of the grant of the patent: **27.11.2024**
(21) Application number: 21759925.7
(22) Date of filing: 24.02.2021
(51) Int. Cl.: C09D 171/02, A61L 27/34, A61L 27/54, C08G 75/045, A61L 31/16, A61L 31/14

(54) **CROSSLINKED POLY(LACTIDE-CO-GLYCOLIDE) (PLGA)-DIMETHACRYLATE COATINGS AND METHODS OF USE**
VERNETZTE POLY(LACTID-CO-GLYCOLID)(PLGA)-DIMETHACRYLAT-BESCHICHTUNGEN UND VERFAHREN ZUR VERWENDUNG
REVÊTEMENTS DE DIMÉTHACRYLATE DE POLY(LACTIDE-CO-GLYCOLIDE) (PLGA) RÉTICULÉS ET LEURS PROCÉDÉS D'UTILISATION

(30) Priority: 26.02.2020 US 202062981696 P
(43) Date of publication of application: 04.01.2023
(73) Proprietor: The Regents of the University of California, Oakland, California 94607-5200 (US)
(72) Inventor: BERNTHAL, Nicholas, Los Angeles, California 90095-7191 (US); XI, Weixian, Los Angeles, California 90095-7191 (US); SEGURA, Tatiana, Los Angeles, California 90095-7191 (US)
(74) Representative: Lecomte & Partners
(86) International application number: PCT/US2021/019485
(87) International publication number: WO 2021/173727

(56) References cited:
- US-A1- 2009 149 941
- US-A1- 2019 388 583
- No further relevant documents disclosed
- MAKADIA, HK ET AL.: "Poly Lactic-co-Glycolic Acid (PLGA) as Biodegradable Controlled Drug Delivery Carrier", POLYMERS, vol. 3, no. 3, 1 September 2011 (2011-09-01), pages 1377 - 1397, XP055759730, DOI: 10.3390/polym3031377
- DAVE, B: "Bioresorbable Scaffolds: Current Evidences in the Treatment of Coronary Artery Disease", JOURNAL OF CLINICAL AND DIAGNOSTIC RESEARCH, vol. 10, no. 10, October 2016 (2016-10-01), pages OE01 - OE07, XP055849750, DOI: 10.7860/JCDR/2016/21915.8429

## Description

### Technical Field

The technical field generally relates to the synthesis and use of drug-containing crosslinked polymers for the use as coatings on medical devices. In particular, the technical field relates to photo-crosslinked poly(lactide-co-glycolide) (PLGA)-dimethacrylate coatings that incorporate antibiotics that are useful for biomedical implants (e.g., orthopedic implants).

### Background

Orthopedic implant infections persist and cost the U.S. health system more than $8 billion per year in additional expenses, despite best efforts at host modification, environmental sterility, and systemic antibiotic therapy. Current standard of care for local antibiotic delivery is polymethylmethacralate (PMMA) - a biologically inert delivery system with poor elution characteristics that is used for its mechanical properties, with local antibiotic delivery as a beneficial "secondary" function. As an alternative to PMMA, attempts have been made to engineer colloidal-based biodegradable systems with self-assembling block polymers as an alternative. For example, a "smart" implant coating using branched poly(ethylene glycol)-poly(propylene sulfide) (PEG-PPS) polymer has been designed to deliver antibiotics both passively and actively. See Stavrakis et al., In Vivo Efficacy of a "Smart" Antimicrobial Implant Coating, Journal of Bone and Joint Surgery, 98(14): 1183-89 (2016). However, these coatings have been labor-intensive to manufacture and thus required assembly prior to the operating room.

WO 2018/144481 A1 discloses a method of coating one or more surfaces of a medical device comprising: providing a mixture of multi-arm poly(ethylene glycol) (PEG), polyallyl mercaptan (PAM), a photoinitiator, an organic solvent, and one or more drugs, medicaments, or pharmaceutical compounds; applying the mixture to one or more surfaces of the medical device; and irradiating the mixture with polymerizing light to form a PEG-PAM coating on the one or more surfaces of the medical device containing the one or more drugs, medicaments, or pharmaceutical compounds.

A persistent problem with polymer-based implant coatings has been the initial burst release of the antibiotics, which cannot provide enough elution time to keep the concentration above the minimum inhibitory concentration (MIC). The MIC is the lowest concentration of a drug (e.g., antibiotic) which prevents growth of bacteria. It is desirous to have coatings for medical devices and other biomedical devices that can be tailored or tuned to release the drug or therapeutic compound (e.g., antibiotic) over an extended period of time and above the MIC level. There thus is a need for additional polymer-based coatings for use with medical devices.

### Summary

Disclosed herein is a polymeric delivery coating for the delivery of medicinal or therapeutic compounds such as antibiotics over an extended period of time and at levels or concentrations that exceed the MIC. In one embodiment, an antibiotic such as vancomycin is encapsulated in a photo-crosslinked poly(lactide-co-glycolide) (PLGA) dimethacrylate coating. The drug release profile of this coating was studied and the initial burst was reduced by photo-crosslinking. Due to photo-crosslinking, an additional diffusional resistance was created, which prevented easy diffusion of the drug into the release medium. Moreover, the time required for this coating process is very quick (e.g., around 5 minutes) and makes it compatible for this coating to be applied in the operating room. This sustained-release of antibiotics is expected to prevent the bacterial infection on implant surface. The coatings may be used to incorporate one or more drugs or pharmaceutical compounds in the presences of a photoinitiator and appropriate light source (e.g., ultraviolet or UV light source) to form crosslinked PLGA dimethacrylate coatings that may be formed *in situ* on, for example, the surface of a medical device. The coating permits medical devices such as orthopedic implants to remain sterile leaving industry warehouses and provides surgeons the versatility to apply an antimicrobial coating (or other drug) in the operating room. A variety of modalities may be used to apply the drug-containing coating. For example, the coating may be applied as an aerosol such as a spray. The coating may also be applied as a paint using an applicator such as a brush or the like (or dipped). The applied coating is then subject to illumination from an appropriate wavelength-emitting light source (e.g., ultraviolet light source) for photo-crosslinked embodiments. The coating system described herein provides a medical device coating that has consistent and appropriate release kinetics and allows patient specific tailoring of antibiotics.

In one embodiment, the process of coating the medical device and polymerizing the coating may be performed by the surgeon or physician inside the medical operating room or the like. For example, a mixture or solution containing the prepolymer solution (e.g., PLGA dimethacrylate, dimethylolpropionic acid (DMPA) (photoinitiator), a drug or therapeutic agent, and a solvent such as dichloromethane) may be provided for use by the surgeon. This mixture is then applied to the medical device and exposed to polymerizing light (e.g., ultra-violet light). The solvent evaporates and does not remain in the coating after application. The surgeon or physician may add the desired drug(s) to this mixture or solution prior to application. Alternatively, the prepolymer solution may already be pre-mixed with the drug(s).

The invention is directed to a method of coating one or more surfaces of a medical device, according to claim 1 and including providing a mixture of poly(lactide-co-glycolide) (PLGA) dimethacrylate, a photoinitiator, an organic solvent, and one or more drugs, medicaments, or pharmaceutical compounds. The mixture is applied to one or more surfaces of the medical device. The mixture on the medical device is then irradiated with polymerizing light to form a photo-crosslinked PLGA dimethacrylate coating on the one or more surfaces of the medical device containing the one or more drugs, medicaments, or pharmaceutical compounds. The now-coated medical device can be implanted. Coating may also take place is an implant that has already been implanted (e.g., *in situ* application of the coating). Further developments of the invention are according to dependent claims 2-6.

The invention is also directed to a method of coating one or more surfaces of a medical device, according to claim 7 and including providing a mixture of poly(lactide-co-glycolide) (PLGA) dimethacrylate, an organic solvent, and one or more drugs, medicaments, or pharmaceutical compounds. The mixture is applied to one or more surfaces of the medical device. The mixture is then crosslinked to form a crosslinked PLGA dimethacrylate coating on the one or more surfaces of the medical device containing the one or more drugs, medicaments, or pharmaceutical compounds, wherein crosslinking is initiated by exposure to a thermal initiator. Further developments of the invention are according to dependent claims 8-12.

The invention is also directed to a medical device containing a coating material, according to claim 13. Further developments of the invention are according to dependent claims 14-16.

### Brief Description of the Drawings

FIG. 1A schematically illustrates a process for applying or forming a photo-crosslinked PLGA coating on medical device or implant according to one embodiment.
FIG. 1B illustrates a process for applying or forming a crosslinked PLGA coating on medical device or implant according to another embodiment.
FIG. 2 illustrates a graph of concentration of vancomycin in the crosslinked PLGA coating on a titanium pin immersed in 200 µl PBS over a number of days (*in vitro* testing).
FIG. 3 illustrates the measured bioluminescent signals of a number of titanium pins (PLGA only (no drug), uncoated titanium (Ti) pin, sterile, and PLGA+vancomycin) using the Xenogen *in vivo* imaging system (Xenogen Corporation, Alameda, CA). The PLGA/Vancomycin group showed very great eradication of infection. The bioluminescent signal is almost the same as the sterile group.
FIG. 4A illustrates the colony forming unit (CFU) measurements in soft tissue (Tissue CFU) post-operative day (POD) twenty-one (21).
FIG. 4B illustrates the CFU measurements in the implant at POD 21. The results of FIGS. 4A and 4B confirmed significant inhibition of bacterial growth both on the implant and in the soft tissue after post-operative day (POD) twenty-one (21). PLGA + Vancomycin results (PLGA + VANC) are similar to sterile pin.

### Detailed Description of Illustrated Embodiments

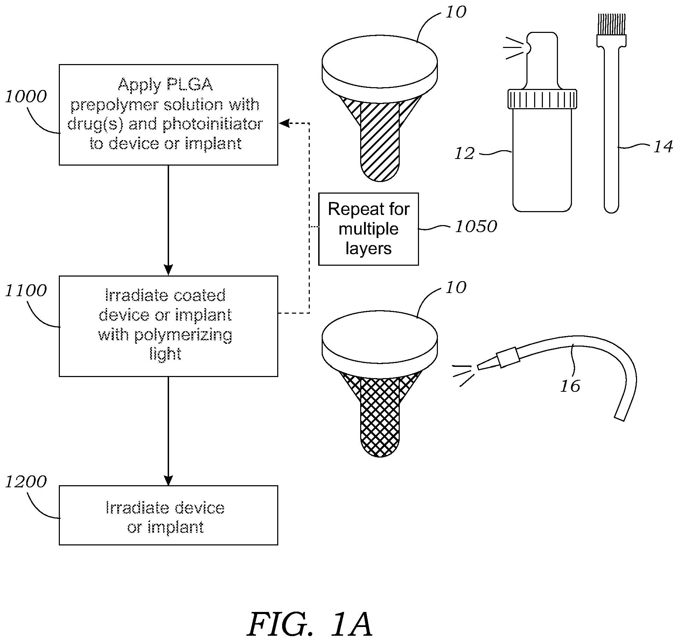

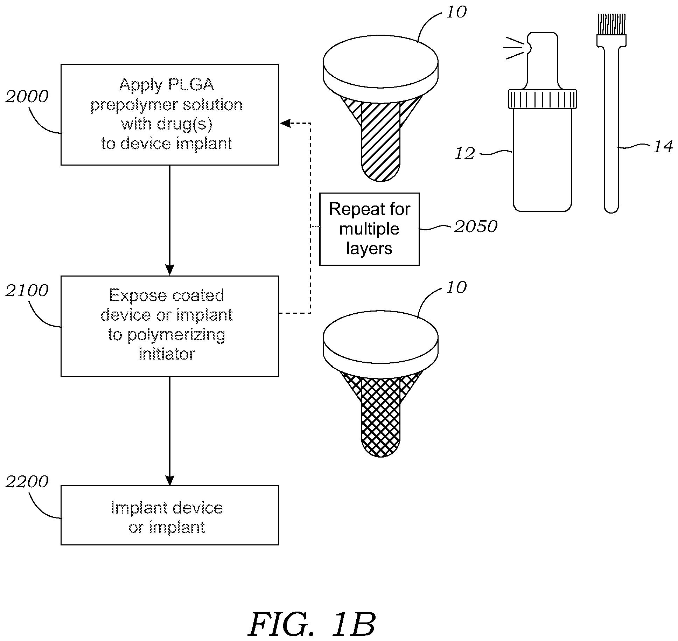

In one embodiment, a drug delivery platform is disclosed that is formed from photo-crosslinked PLGA and in particular photo-crosslinked PLGA dimethacrylate that is used to coat one or more surfaces of a medical device or implant 10 (FIGS. 1A, 1B). In some embodiments, the crosslinked PLGA utilizes acrylates (e.g., PLGA dimethacrylate). PLGA may also be used with acrylamides. In some embodiments, the coating is degradable over time (e.g., PLGA is degradable by the body). The photo-crosslinked PLGA system may be used for the delivery of medicinal or therapeutic compounds such as antibiotics to living tissue. The polymeric delivery platform, in one embodiment, is coated *in situ* on one or more surfaces of a medical device or implant 10 that is to be used in a mammalian subject. In one preferred embodiment, a prepolymer solution of PLGA dimethacrylate in the presence of an initiator compound (e.g., photoinitiator) and a solvent such as dichloromethane is mixed with one or more drugs, medicaments, or pharmaceutical compounds (e.g., a solution containing the same). The mixture is exposed to an initiator source such as light (in a preferred embodiment), heat, or other initiator/stimulus for aiding in free radical polymerization reactions to polymerize the copolymer (e.g., redox initiator). In one embodiment, the initiator compound is a photoinitiator (e.g., dimethylolpropionic acid or 2,2-Dimethoxy-2-phenylacetophenone (DMPA)) and the mixture is first applied to one or more surfaces of a medical device or implant 10 and is then exposed to an appropriate polymerizing light source (e.g., ultraviolet light source or visible light source) to form a crosslinked PLGA coating layer or multiple layer(s) that is/are formed *in situ* on the medical device or implant 10.

While a photoinitiator is described herein, in other embodiments, other initiators that crosslink the PLGA may also be used. For example, redox initiator that is generated in response to applied heat (i.e., thermal initiator) may be used to crosslink the PLGA dimethacrylate. Examples of such redox initiators include, for example, azo-compounds or peroxides. Commercially available free radical regulators are also available for free radical polymerization applications. An example is the BlocBuilder^{®} MA (Arkema) regulator that is an alkoxyamine joining a methacrylate acid-based radical initiating species along with a reaction controller molecule. The application of heat liberates the radical initiator which can then be used crosslink the PLGA dimethacrylate. It should be appreciated that other redox initiators may be used beyond those specifically disclosed herein. In addition, a combination of light and heat may be used in other embodiments. For example, localized heating in response to light irradiation may liberate thermal initiators to aid in polymerization.

The PLGA coating permits medical devices or implants 10 to remain in a sterile state prior to use and provides surgeons the versatility to customize the drug, medicament, or pharmaceutical compound-containing coating on a medical device or implant 10 (or applied on or within the patient directly within the operating room in other applications). In some embodiments, the drug, medicament, or pharmaceutical compound includes one or more antimicrobial agents or drugs that can be applied directly to all or part of a medical device or implant 10. The choice of antimicrobial agent used with the PLGA coating may be specifically tailored to the patient's needs. Of course, in other embodiments, the PLGA coating may be applied the device or implant 10 prior to use in the operating rooms (e.g., at the site of manufacturing).

FIG. 1A schematically illustrates one embodiment of a method of coating one or more surfaces of a medical device or implant 10. The medical device or implant 10 may include, by way of illustration and not limitation, an orthopedic implant, a spinal implant, a joint replacement device, pacemakers, insulin pumps, surgical pins, screws, or other surgical hardware. Typically, the coating is applied to a metallic surface of the medical device or implant 10 such as titanium or stainless steel but it should be appreciated that the coating and method of use is not limited to the particular composition of the substrate on which the coating resides. For example, it may also be applied to polymer or ceramic surfaces. In one particular example, a prepolymer solution (e.g., 40 mg PLGA dimethacrylate, 1 mg DMPA in 400 µL dichloromethane (DCM)) was combined with a drug, medicament, or pharmaceutical component (e.g., vancomycin solution (8mg in 20ul PBS)) and stored in a delivery device 12. Other organic solvents that may be used include chloroform and dimethyl sulfide (DMS). The amount of organic solvent may vary but is typically under 10 % weight basis (e.g., 6% by weight). In the example described herein, the drug that is incorporated into the PLGA coating is the antibiotic vancomycin. Other antibiotics that could also be used include those that belong to antibacterial families including glicopeptides, cephalosporins, aminoglicosydes, macrolids, oxazolidinones, quinolones, polymixins, sulfonamides, tetracyclines and penicillins. Examples include daptomicin, tigecycline, telavancin, chloramphenicol, bacitracin, rifampin, ethambutol, streptomycin, isoniazid. Different solvents appropriate for these antibiotics may be needed including non-organic solvents.

It should be appreciated that other drugs, medicaments, and pharmaceutical compounds may be loaded within the PLGA coating. Because of the use of the organic solvent (in the case of vancomycin), the drugs, medicaments, or pharmaceutical compounds should generally be dissolvable or soluble in organic solvents. It should also be appreciated that more than one type of drug, medicament, or pharmaceutical compounds may be carried by the coating formed by the methods described herein.

In the operating room or other surgical setting, the surgeon or physician is provided with or assembles the mixture of PLGA prepolymer solution and the drug, medicament, or pharmaceutical compound (or the mixture is already prepared). The amount of drug, medicament, or pharmaceutical compound that is added may vary. A typical concentration of the drug, medicament, or pharmaceutical compound is generally within or below about 10-20 mg/mL of prepolymer mixture.

This prepolymer/drug mixture may be added (if not already present therein) to a delivery device 12 such as spray bottle or the like that can be used to spray a coating onto the medical device or implant 10 (other delivery devices 12 such as air-brush type applicators may also be used). The spray bottle 12 may actuated to apply a generally uniform coating of the mixture onto one or more surfaces of the medical device or implant 10 as seen in operation 1000 of FIG. 1A. Alternatively, medical device or implant 10 may be coated with the mixture with an applicator 14 such as a brush. As yet another alternative, the medical device or implant 10 may be dipped into the mixture and removed. Next, as seen in operation 1100, the medical device or implant 10 is irradiated with light from a light source 16 (e.g., visible or UV light depending on the initiator) to polymerize the PLGA polymer directly on one or more surfaces of the medical device or implant 10. The light source 16 may include a light, gun, wand, or other device that is typically found in hospitals and other medical settings and is used to illuminate a surface with light. The coating is illuminated for a short period of time (e.g., less than 5 minutes) to properly form the crosslinked PLGA polymer with the drug(s) contained therein. After applying the coating to the medical device or implant 10 some amount of time may elapse to enable the solvent to evaporate prior to illumination. This optional waiting period may last a few minutes. Thus, polymerization takes place *in situ* on the surface of the medical device or implant 10.

As an alternative to photo-crosslinking, in other embodiments a different redox initiator may be used to cross-link the pre-polymer solution mixture (FIG. 1B). For example, the coated medical device or implant 10 may be subject to an elevated temperature to initiate cross-linking using a thermal initiator. This may be accomplished with a heat gun or the like or placing the medical device or implant 10 in an oven or heater. In yet another alternative, cross-linking may be accomplished by exposing the coated medical device or implant 10 to one or more chemical reagents to initiate polymerization of the copolymer. This may be included in a separate delivery device 12 (e.g., separate spray bottle) that is used to apply the chemical reagent(s) onto the medical device or implant 10. Additional methods of exposure may be used such as dipping, use of an applicator 14, or the like.

As seen in FIG. 1A, multiple layers may optionally be coated on the surface of the medical device or implant 10 as seen by operation 1050. For multiple layers, the pre-polymer/drug mixture is applied and then subject to cross-linking (e.g., irradiation with cross-linking light) followed by application of another layer of the mixture followed by irradiation. This can proceed for a number of cycles to create the desired thickness or number of layers on the medical device or implant 10.

FIG. 1B illustrates an alternative method of coating one or more surfaces of a medical device or implant 10. The PLGA prepolymer solution and drug(s) are applied to the medical device or implant 10 as seen in operation 2000. This may be applied as described previously. Next, in operation 2100, the coated device or implant 10 is then exposed to a polymerizing initiator (e.g., thermal initiator). This may include exposing the device or implant 10 to an environmental condition such as elevated temperature or exposure to a polymerizing initiator or agent. Optionally, this process may be repeated a number of times as seen in operation 2050. Once the desired layer (or multiple layers) are formed, the medical device or implant 10 may then be implanted as seen in operation 2200.

In some embodiments, only a portion of the surface of the medical device or implant 10 is coated. For example, it may be preferred that only those surfaces that are in contact with bone or bodily tissue are to be coated. Those surfaces that serve as contact or articulating surfaces for other components may not be coated. The coating system described herein may be used with any number of medical devices or implants 10 but has particular suitability to joint replacement devices. The coating system may be used also with orthopedic trauma implants or spinal implants. Other implantable medical devices may also be coated such as pacemakers, insulin pumps, and the like. The surfaces that are coated are typically metal such as titanium, stainless steel, cobalt-chrome although other materials may also be coated. These include plastics, ceramics, as well as allograft cadaver bone.

In the embodiments described herein, the initiator is a photoinitiator (e.g., DMPA) which requires activation by light. It should be understood that in other embodiments, a heat applicator may be used to apply heat when the initiator is a thermal initiator. This may include a heat gun, oven, warming device, or the like. In other embodiments, for example, where the redox initiator is one or more chemical agents, there may be no need for a separate stimulation device. The process from start to finish is may be accomplished relatively quickly, for example, under 10 minutes (e.g., around 5 minutes) depending on the number and size of surfaces to be coated. Finally, as seen in operations 1200, 2200, the medical device or implant 10 is implanted in the subject.

### Experimental

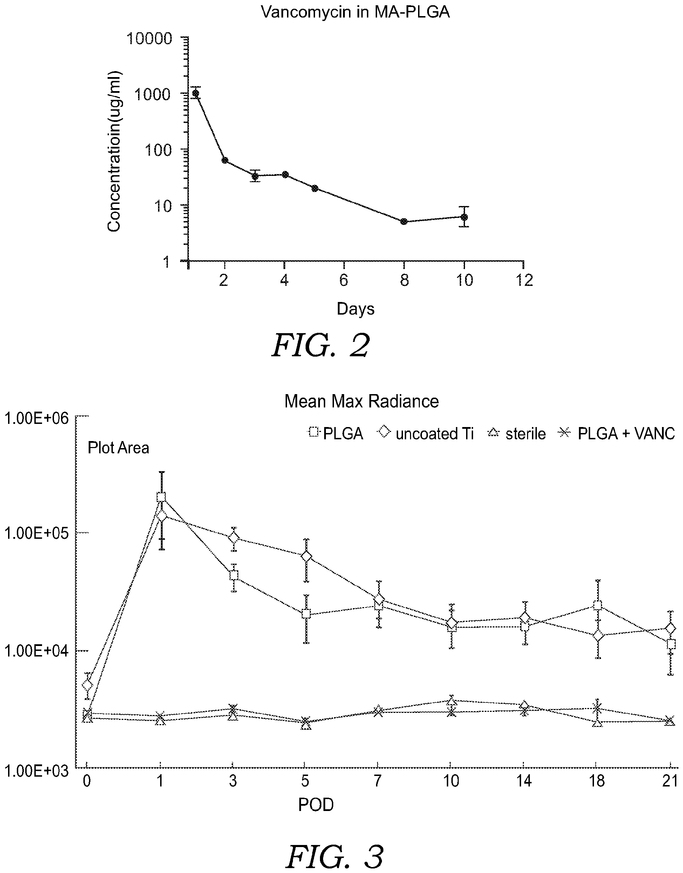

Titanium pins (representative of a medical device or implant 10) were dipped in a prepolymer solution (40 mg PLGA dimethacrylate, 1 mg DMPA in 400 µl dichloromethane) combined with vancomycin solution (8mg in 20 µl PBS), and then the pin was taken out from the solution and irradiated under UV light for 5 min. The coated pin was then immersed in 200 µl PBS and the elution buffer was daily refreshed for *in vitro* release testing. The concentration was determined by UV absorption of vancomycin at 230 nm. FIG. 2 illustrates a graph of the concentration of vancomycin in crosslinked PLGA dimethacrylate (MA-PLGA). The initial burst or release of vancomycin was reduced by photo-crosslinking. Due to photo-crosslinking, an additional diffusional resistance is created, which prevented easy diffusion of the drug into the release medium; thereby maintaining the vancomycin in the coating on the pin. In one preferred embodiment, the level of antibiotic in the coating or immediately adjacent to the coating remains above the MIC for a period of time. For example, the level or concentration of the antibiotic may remain above the MIC level for a plurality of days or even weeks.

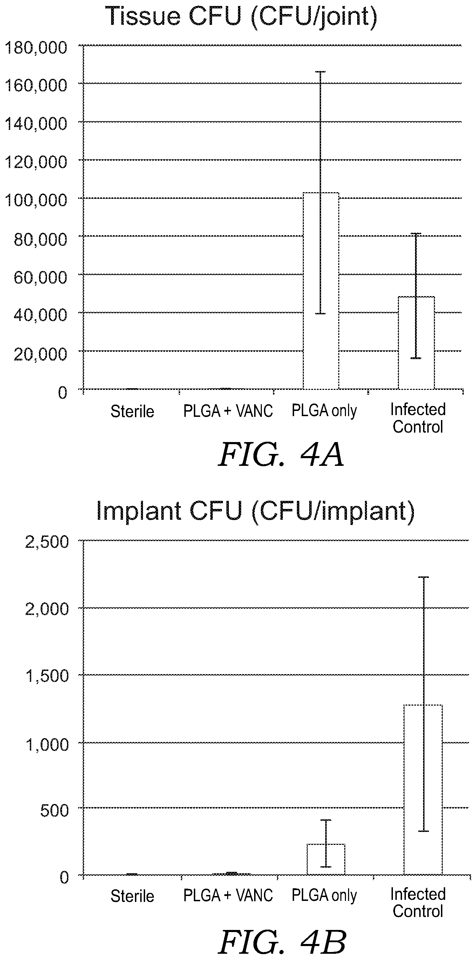

Pins coated with PLGA-dimethacrylate were also evaluated *in vivo.* Specifically, the implant coating technology was investigated whether it could effectively prevent infection after periprosthetic joint infection (PJI) using a mouse model of knee PJI previously developed in the lab. Briefly, a titanium pin (0.8 mm) with the crosslinked PLGA-dimethacrylate polymer coating is placed retrograde, from the knee joint into the femoral canal. Bioluminescent *Staphylococcus aureus* Xen36 is used to inoculate the intra-articular portion of the metal pin in the joint space to induce the formation of a controlled infection that can be non-invasively quantified using the Xenogen *in vivo* imaging system (Xenogen Corporation, Alameda, CA). The *in vivo* assay employed bioluminescent Staphylococcus aureus Xen36 strain that naturally produces a blue-green light emitted only by metabolically active bacteria as an "indicator" that reflects bacterial infections. The PLGA/Vancomycin group showed superior results for the eradication of infection. The bioluminescent signal from the PLGA/Vancomycin group (PLGA + VANC) is almost the same as the sterile group (FIG. 3). PLGA only has almost same signal as the control (no coating group) which means PLGA only cannot provide enough protection of the implant 10. Also, the CFUs measurements illustrated in FIGS. 4A and 4B also confirm significant inhibition of bacterial growth both on the implant 10 and in the soft tissue (peri-implant tissue) after post-operative day (POD) 21.

Note that the eluting properties of the PLGA coating can be adjusted or tuned depending on the desired properties of the coated device or implant 10. In some embodiments, the drug, medicament, or pharmaceutical may be eluted in a linear fashion over time (this may be a slow release or a rapid release). In other embodiments, an increase or spike (e.g., bolus) in elution may be desired either right after implantation or several days or weeks after implementation. The PLGA elution kinetics may be modified for the appropriate application. The release kinetics can be tuned, in one embodiment, by adjusting the degree of crosslinking (e.g., by adjusting the time of the initiator is applied - e.g., light). A higher degree of crosslinking will generally result in a slower release profile. Tuning may also be accomplished by varying the degree of crosslinking in multiple layers that are formed on the device or implant 10. The biodegradation rate of the PLGA coating may also be tuned. This may be accomplished by tuning the ratio of lactic:glycolic acid in the PLGA coating. For example, higher lactic acid content generally increases the degradation rate of PLGA.

While embodiments of the present invention have been shown and described, various modifications may be made without departing from the scope of the present invention. The invention, therefore, should not be limited, except to the following claims.

## Claims

1. A method of coating one or more surfaces of a medical device (10) comprising:
providing a mixture of poly(lactide-co-glycolide) (PLGA) dimethacrylate, a photoinitiator, an organic solvent, and one or more drugs, medicaments, or pharmaceutical compounds;
applying (1000) the mixture to one or more surfaces of the medical device (10); and
irradiating (1100) the mixture with polymerizing light to form a photo-crosslinked PLGA dimethacrylate coating on the one or more surfaces of the medical device (10) containing the one or more drugs, medicaments, or pharmaceutical compounds.

2. The method of claim 1, wherein the mixture is applied to one or more surfaces of the medical device (10) by spraying, painting, or dipping.

3. The method of claim 1, wherein the medical device (10) comprises one or more metallic surfaces that are coated with the photo-crosslinked PLGA dimethacrylate coating.

4. The method of claim 1, wherein the medical device (10) comprises one of an orthopedic implant, a spinal implant, a joint replacement device, a pacemaker, an insulin pumps, a surgical pin, a screw, or other surgical hardware.

5. The method of claim 1, wherein the one or more drugs, medicaments, or pharmaceutical compounds comprises an antibiotic.

6. The method of claim 5, wherein the antibiotic comprises vancomycin.

7. A method of coating one or more surfaces of a medical device (10) comprising:
providing a mixture of poly(lactide-co-glycolide) (PLGA) dimethacrylate, an organic solvent, and one or more drugs, medicaments, or pharmaceutical compounds;
applying (2000) the mixture to one or more surfaces of the medical device (10); and
crosslinking (2100) the mixture to form a crosslinked PLGA dimethacrylate coating on the one or more surfaces of the medical device (10) containing the one or more drugs, medicaments, or pharmaceutical compounds, wherein crosslinking is initiated by exposure to a thermal initiator.

8. The method of claim 7, wherein the mixture is applied to one or more surfaces of the medical device by spraying, painting, or dipping.

9. The method of claim 7, wherein the medical device (10) comprises one or more metallic surfaces that are coated with the crosslinked PLGA dimethacrylate coating.

10. The method of claim 7, wherein the medical device (10) comprises one of an orthopedic implant, a spinal implant, a joint replacement device, a pacemaker, an insulin pumps, a surgical pin, a screw, or other surgical hardware.

11. The method of claim 7, wherein the one or more drugs, medicaments, or pharmaceutical compounds comprises an antibiotic.

12. The method of claim 7, wherein the antibiotic comprises vancomycin.

13. A medical device (10) containing a coating material disposed on one or more surfaces thereof, the coating material comprising:
poly(lactide-co-glycolide) (PLGA) dimethacrylate containing one or more drugs, medicaments, or pharmaceutical compounds therein and a polymerizing initiator.

14. The medical device (10) of claim 13, wherein the one or more drugs, medicaments, or pharmaceutical compounds comprises one or more antibiotics.

15. The medical device (10) of claim 13, wherein the coating material comprises multiple layers.

16. The medical device of claim 13, wherein the polymerizing initiator is one of a photoinitiator, thermal initiator, or chemical initiator.

## Patentansprüche

1. Ein Verfahren zum Beschichten einer oder mehrerer Oberflächen einer medizinischen Vorrichtung (10), das Folgendes umfasst:
Bereitstellen einer Mischung aus Poly(lactid-co-glycolid)-(PLGA)-Dimethacrylat, einem Photoinitiator, einem organischen Lösungsmittel und einem oder mehreren Arzneimitteln, Medikamenten oder pharmazeutischen Verbindungen;
Auftragen (1000) der Mischung auf eine oder mehrere Oberflächen der medizinischen Vorrichtung (10); und
Bestrahlen (1100) der Mischung mit polymerisierendem Licht, um eine photovernetzte PLGA-Dimethacrylat-Beschichtung auf der einen oder den mehreren Oberflächen der medizinischen Vorrichtung (10) zu bilden, die das eine oder die mehreren Arzneimittel, Medikamente oder pharmazeutischen Verbindungen enthält.

2. Das Verfahren nach Anspruch 1, wobei die Mischung auf eine oder mehrere Oberflächen der medizinischen Vorrichtung (10) durch Sprühen, Streichen oder Tauchen aufgebracht wird.

3. Das Verfahren nach Anspruch 1, wobei die medizinische Vorrichtung (10) eine oder mehrere metallische Oberflächen umfasst, die mit der photovernetzten PLGA-Dimethacrylatbeschichtung beschichtet sind.

4. Das Verfahren nach Anspruch 1, wobei die medizinische Vorrichtung (10) ein orthopädisches Implantat, ein Wirbelsäulenimplantat, eine Gelenkersatzvorrichtung, einen Herzschrittmacher, eine Insulinpumpe, einen chirurgischen Stift, eine Schraube oder andere chirurgische Hardware umfasst.

5. Das Verfahren nach Anspruch 1, wobei das eine oder die mehreren Arzneimittel, Medikamente oder pharmazeutischen Verbindungen ein Antibiotikum umfassen.

6. Das Verfahren nach Anspruch 5, wobei das Antibiotikum Vancomycin umfasst.

7. Ein Verfahren zum Beschichten einer oder mehrerer Oberflächen einer medizinischen Vorrichtung (10), das Folgendes umfasst:
Bereitstellen einer Mischung aus Poly(lactid-co-glycolid)-(PLGA)-Dimethacrylat, einem organischen Lösungsmittel und einem oder mehreren Arzneimitteln, Medikamenten oder pharmazeutischen Verbindungen;
Auftragen (2000) der Mischung auf eine oder mehrere Oberflächen der medizinischen Vorrichtung (10); und
Vernetzen (2100) der Mischung, um eine vernetzte PLGA-Dimethacrylat-Beschichtung auf der einen oder den mehreren Oberflächen der medizinischen Vorrichtung (10) zu bilden, die das eine oder die mehreren Arzneimittel, Medikamente oder pharmazeutischen Verbindungen enthält, wobei die Vernetzung durch Einwirkung eines thermischen Initiators eingeleitet wird.

8. Das Verfahren nach Anspruch 7, wobei die Mischung auf eine oder mehrere Oberflächen der medizinischen Vorrichtung durch Sprühen, Streichen oder Tauchen aufgebracht wird.

9. Das Verfahren nach Anspruch 7, wobei die medizinische Vorrichtung (10) eine oder mehrere metallische Oberflächen umfasst, die mit der vernetzten PLGA-Dimethacrylatbeschichtung beschichtet sind.

10. Das Verfahren nach Anspruch 7, wobei die medizinische Vorrichtung (10) eines der folgenden Elemente umfasst: ein orthopädisches Implantat, ein Wirbelsäulenimplantat, eine Gelenkersatzvorrichtung, einen Herzschrittmacher, eine Insulinpumpe, einen chirurgischen Stift, eine Schraube oder andere chirurgische Hardware.

11. Das Verfahren nach Anspruch 7, wobei das eine oder die mehreren Arzneimittel, Medikamente oder pharmazeutischen Verbindungen ein Antibiotikum umfassen.

12. Das Verfahren nach Anspruch 7, wobei das Antibiotikum Vancomycin umfasst.

13. Eine medizinische Vorrichtung (10), die ein Beschichtungsmaterial enthält, das auf einer oder mehreren Oberflächen davon angeordnet ist, wobei das Beschichtungsmaterial Folgendes umfasst:
Poly(lactid-co-glycolid)-(PLGA)-Dimethacrylat, das ein oder mehrere Arzneimittel, Medikamente oder pharmazeutische Verbindungen enthält, und einen Polymerisationsinitiator.

14. Die medizinische Vorrichtung (10) nach Anspruch 13, wobei das eine oder die mehreren Arzneimittel, Medikamente oder pharmazeutischen Verbindungen ein oder mehrere Antibiotika umfassen.

15. Die Medizinische Vorrichtung (10) nach Anspruch 13, wobei das Beschichtungsmaterial mehrere Schichten umfasst.

16. Die medizinische Vorrichtung nach Anspruch 13, wobei der Polymerisationsinitiator einer der folgenden ist: Photoinitiator, thermischer Initiator oder chemischer Initiator.

## Revendications

1. Un procédé de revêtement d'une ou plusieurs surfaces d'un dispositif médical (10) comprenant les étapes consistant à :
fournir un mélange de diméthacrylate de poly(lactide-co-glycolide) (PLGA), un photo-initiateur, un solvant organique et un ou plusieurs médicaments ou composés pharmaceutiques ;
appliquer (1000) le mélange sur une ou plusieurs surfaces du dispositif médical (10) ; et
irradier (1100) le mélange avec une lumière polymérisante pour former un revêtement de diméthacrylate de PLGA photoréticulé sur une ou plusieurs surfaces du dispositif médical (10) contenant un ou plusieurs médicaments, substances actives ou composés pharmaceutiques.

2. Le procédé selon la revendication 1, dans lequel le mélange est appliqué sur une ou plusieurs surfaces du dispositif médical (10) par pulvérisation, peinture ou trempage.

3. Le procédé selon la revendication 1, dans lequel le dispositif médical (10) comprend une ou plusieurs surfaces métalliques qui sont recouvertes d'un revêtement de diméthacrylate de PLGA photoréticulé.

4. Le procédé selon la revendication 1, dans lequel le dispositif médical (10) comprend un implant orthopédique, un implant rachidien, un dispositif de remplacement articulaire, un stimulateur cardiaque, une pompe à insuline, une broche chirurgicale, une vis ou tout autre matériel chirurgical.

5. Le procédé selon la revendication 1, dans lequel un ou plusieurs médicaments ou composés pharmaceutiques comprennent un antibiotique.

6. Le procédé selon la revendication 5, dans lequel l'antibiotique comprend la vancomycine.

7. Un procédé de revêtement d'une ou plusieurs surfaces d'un dispositif médical (10) comprenant les étapes consistant à :
fournir un mélange de diméthacrylate de poly(lactide-co-glycolide) (PLGA), un solvant organique et un ou plusieurs médicaments, substances actives ou composés pharmaceutiques ;
appliquer (2000) le mélange sur une ou plusieurs surfaces du dispositif médical (10) ; et
réticuler (2100) le mélange pour former un revêtement de diméthacrylate de PLGA réticulé sur une ou plusieurs surfaces du dispositif médical (10) contenant un ou plusieurs médicaments ou composés pharmaceutiques, la réticulation étant initiée par l'exposition à un initiateur thermique.

8. Le procédé selon la revendication 7, dans lequel le mélange est appliqué sur une ou plusieurs surfaces du dispositif médical par pulvérisation, peinture ou trempage.

9. Le procédé selon la revendication 7, dans lequel le dispositif médical (10) comprend une ou plusieurs surfaces métalliques recouvertes d'un revêtement de diméthacrylate de PLGA réticulé.

10. Le procédé selon la revendication 7, dans lequel le dispositif médical (10) comprend un implant orthopédique, un implant rachidien, un dispositif de remplacement articulaire, un stimulateur cardiaque, une pompe à insuline, une broche chirurgicale, une vis ou tout autre matériel chirurgical.

11. Le procédé selon la revendication 7, dans lequel un ou plusieurs médicaments ou composés pharmaceutiques comprennent un antibiotique.

12. Le procédé selon la revendication 7, dans lequel l'antibiotique comprend la vancomycine.

13. Un dispositif médical (10) contenant un matériau de revêtement disposé sur une ou plusieurs de ses surfaces, le matériau de revêtement comprenant :
du diméthacrylate de poly(lactide-co-glycolide) (PLGA) contenant un ou plusieurs médicaments, substances actives ou composés pharmaceutiques et un initiateur de polymérisation.

14. Le dispositif médical (10) selon la revendication 13, dans lequel un ou plusieurs médicaments, substances actives ou composés pharmaceutiques comprennent un ou plusieurs antibiotiques.

15. Le dispositif médical (10) selon la revendication 13, dans lequel le matériau de revêtement comprend plusieurs couches.

16. Le dispositif médical selon la revendication 13, dans lequel l'initiateur de polymérisation est un photo-initiateur, un initiateur thermique ou un initiateur chimique.
